(19) Europäisches Patentamt
European Patent Office
Office européen des brevets

(11) Veröffentlichungsnummer : **0 022 079**
**B1**

(12) # EUROPÄISCHE PATENTSCHRIFT

(45) Veröffentlichungstag der Patentschrift : **18.01.84**

(51) Int. Cl.$^3$ : **C 07 D 211/58**, C 08 K 5/34

(21) Anmeldenummer : **80810201.6**

(22) Anmeldetag : **16.06.80**

(54) **Polyalkylpiperidyl-harnstoffe, ihre Verwendung als Stabilisatoren und die damit stabilisierten Polymeren.**

(30) Priorität : **21.06.79 CH 5807/79**

(43) Veröffentlichungstag der Anmeldung : **07.01.81 Patentblatt 81/01**

(45) Bekanntmachung des Hinweises auf die Patenterteilung : **18.01.84 Patentblatt 84/03**

(84) Benannte Vertragsstaaten :
**CH DE FR GB IT LI**

(56) Entgegenhaltungen :
**DE-A- 2 040 975**
**DE-A- 2 349 962**
**DE-A- 2 621 870**
**DE-A- 2 642 461**
**DE-A- 2 821 579**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Patentabteilung Postfach**
**CH-4002 Basel (CH)**

(72) Erfinder : **Karrer, Friedrich, Dr.**
**Rebbergstrasse 5**
**CH-4800 Zofingen (CH)**

**EP 0 022 079 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Polyalkylpiperidyl-harnstoffe, ihre Verwendung als Stabilisatoren, und die damit stabilisierten Polymeren

Die Erfindung betrifft neue substituierte Harnstoffe, die mindestens einen Polyalkylpiperidinrest als Substituenten haben und die wertvolle Stabilisatoren für Kunststoffe sind, insbesondere gegen deren Schädigung durch Licht.

Harnstoffe mit Polyalkylpiperidin-Substituenten und ihre Verwendung als Stabilisatoren sind bereits bekannt. So beschreibt die DE-OS 2 040 975 Derivate von 4-Amino-2,2,6,6-tetramethyl-piperidin der Formel

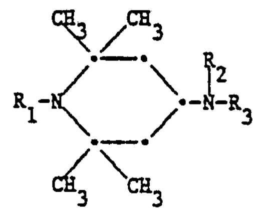

worin $R_1$ H oder eine Acylgruppe ist, $R_2$ H oder ein einwertiger Rest ist und $R_3$ unter anderem eine Carbamoylgruppe oder N-substituierte Carbamoylgruppe sein kann, wie z. B. —$CONH_2$, —CONHAlkyl, —CONHCyclo-alkyl oder —CONHAryl.

In der DE-OS 2 349 962 wurden ähnliche Verbindungen der folgenden Formel beschrieben,

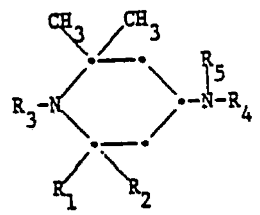

worin $R_1$ und $R_2$ Alkylreste sind, $R_3$ Alkyl, Alkenyl, Alkinyl oder Aralkyl ist, $R_5$ H oder ein einwertiger Rest ist und $R_4$ unter anderem eine N-substituierte oder unsubstituierte Carbamoylgruppe darstellt, beispielsweise —$CONH_2$, —CONHAlkyl, —CONHaryl oder —CONHAralkyl. Die in diesen beiden Patentschriften offenbarten Harnstoffderivate tragen entweder am 1-Stickstoff oder am 3-Stickstoff der Harnstoff-Gruppierung mindestens ein Wasserstoffatom. Beispiele sind der 1-Benzyl-3-(1,2,2,6,6-pentamethyl-4-piperidyl)-harnstoff oder der 1,1-Dimethyl-3-(1,2,2,6,6-pentamethyl-4-piperidyl)-harnstoff. Diese Verbindungen sind gute Lichtschutzmittel für Kunststoffe, wenn diese bei niedrigen Temperaturen verarbeitet werden. Bei höheren Verarbeitungstemperaturen resultiert eine mehr oder weniger merkliche Verfärbung und eine relativ schwache Stabilisator-Wirkung.

Es wurde gefunden, dass Polyalkylpiperidylharnstoffe, deren Harnstoff-Gruppe weder am 1-Stickstoff noch am 3-Stickstoff ein Wasserstoffatom trägt, als Lichtschutzmittel eine wesentlich überlegene Wirkung besitzen. In gewissen Substraten übertrifft ihre Lichtschutzwirkung die der meisten bekannten Lichtschutz-Stabilisatoren, d. h. auch diejenigen von chemisch völlig verschiedener Konstitution.

Die Erfindung betrifft daher Verbindungen der Formel I,

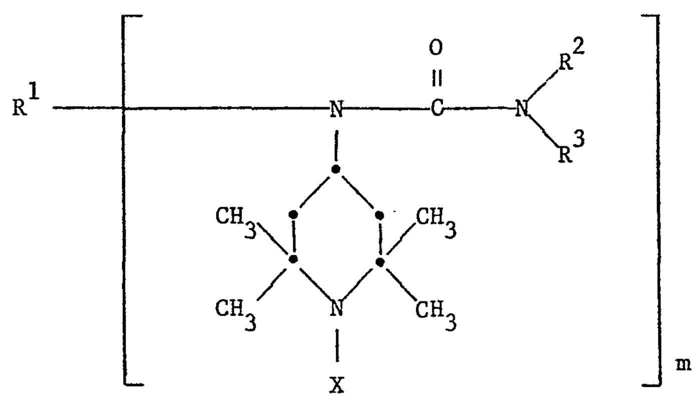

(I)

worin m 1, 2, 3 oder 4 ist, X H, O·, $C_1$-$C_{18}$-Alkyl, $C_3$-$C_5$-Alkenyl, Propargyl, $C_7$-$C_{12}$-Phenylalkyl oder eine Gruppe der Formel —CO—$C_1$-$C_8$-Alkyl, —CO—$C_2$-$C_3$-Alkenyl, —CO—O—$C_1$-$C_{10}$-Alkyl oder —CO—N($C_1$-$C_{10}$-Alkyl)$_2$ bedeutet, $R^1$, wenn m 1 ist, $C_1$-$C_{18}$-Alkyl, $C_3$-$C_5$-Alkenyl, $C_3$-$C_4$-Alkinyl, $C_5$-$C_6$-

Cycloalkyl, $C_7$-$C_{18}$-Aralkyl, $C_6$-$C_{12}$-Aryl oder ein Gruppe der Formel II

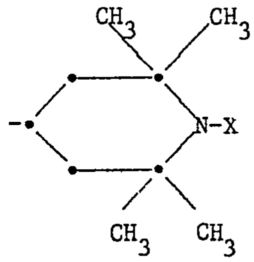

(II)

bedeutet, wenn m 2 ist, $C_2$-$C_{20}$-Alkylen, $C_4$-$C_{12}$-Mono- oder -Dioxaalkylen, eine Gruppe $-(CH_2CH_2\underset{R^4}{\underset{|}{N}})-_nCH_2CH_2-$, $-(CH_2)_3-N(R^5)-(CH_2)_3-$, Cyclohexylen, Xylylen, Hexahydroxylylen, oder eine Gruppe

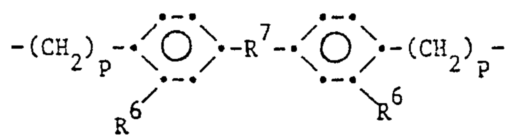

oder

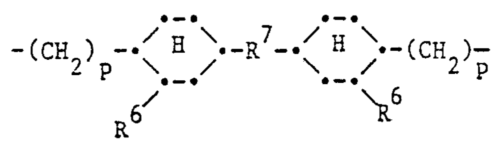

bedeutet, worin $R^4$ Wasserstoff, $C_1$-$C_4$-Alkyl, Allyl, Benzyl oder eine Gruppe

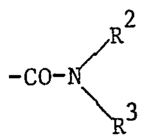

ist, n eine ganze Zahl von 1 bis 5 ist, $R^5$ $C_1$-$C_{12}$-Alkyl, Phenyl oder Cyclohexyl bedeutet, p null oder 1 ist, $R^6$ Wasserstoff oder Methyl ist und $R^7$ $-CH_2-$ oder $>C(CH_3)_2$ bedeutet, wenn m 3 ist, einen Rest

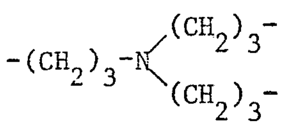

bedeutet, und wenn m 4 ist, einen Rest der Formel

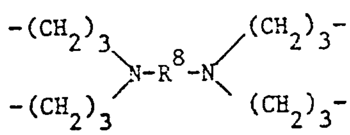

bedeutet, worin $R^8$ $C_2$-$C_{20}$-Alkylen, Cyclohexylen oder Xylylen ist, $R^2$ und $R^3$ unabhängig voneinander $C_1$-$C_{12}$-Alkyl, Cyclohexyl, $C_3$-$C_5$-Alkenyl, Propargyl, $C_7$-$C_{12}$-Phenylalkyl oder $C_6$-$C_{12}$-Aryl bedeuten und $R^2$ auch $C_1$-$C_4$-Alkoxy bedeuten kann oder $R^2$ und $R^3$ zusammen $C_4$-$C_9$-Alkylen oder 3-Oxa-1,5-pentylen bilden.

In Formel I können X und $R^1$ als Alkyl z. B. Methyl, Aethyl, Propyl, Butyl, Pentyl, Isopentyl, Hexyl, Octyl, 2-Aethylhexyl, Dodecyl oder Octadecyl sein. $R^1$, $R^2$, $R^3$ oder X als Alkenyl können z. B. Allyl, Methallyl oder 2-Butenyl sein. $R^1$ als Alkinyl kann z. B. Propargyl oder 2-Butinyl sein.

X, $R^2$ und $R^3$ als Phenylalkyl können z. B. Phenylpropyl, Phenylbutyl oder Phenyläthyl, bevorzugt jedoch Benzyl sein. $R^1$ als Aralkyl kann darüber hinaus auch Naphthylmethyl sein.

Wenn X ein Acylrest $-CO-C_1$-$C_8$-Alkyl ist, so kann dies z. B. Acetyl, Propionyl, Butyryl, Isovalerianyl, Hexanoyl oder Octanoyl sein. X als Alkoxycarbonyl $-CO-O-C_1$-$C_{10}$-Alkyl kann z. B. Methoxycarbonyl, Aethoxycarbonyl oder Octyloxycarbonyl sein. X als Dialkylcarbamoylrest $-CO-N(C_2$-$C_{10}$-$Alkyl)_2$ kann z. B. Dimethylcarbamoyl, Dibutylcarbamoyl oder Dioctylcarbamoyl sein.

$R^1$ als Cycloalkyl kann Cyclopentyl oder Cyclohexyl sein. $R^1$, $R^2$ und $R^3$ als Aryl können z. B. Phenyl, Naphthyl oder Diphenylyl sein.

$R^1$ als Alkylen kann ein verzweigter oder unverzweigter Alkylenrest sein, wie z. B. 1,2-Aethylen, 1,2-Propylen, 1,3-Propylen, 2,2-Diäthyl-1,3-propylen, Tetramethylen, Hexamethylen, Octa-, Deca-, Dodeca- oder Octadecamethylen. $R^1$ als Oxaalkylen kann z. B. 3-Oxa-1,5-pentylen, 4-Oxa-1,7-heptylen oder 4,7-Dioxa-1,10-decylen sein. Wenn $R^2$ und $R^3$ zusammen $C_4$-$C_5$-Alkylen oder 3-Oxapentylen sind, so bilden sie zusammen mit dem N-Atom, an das sie gebunden sind, z. B. einen Pyrrolidin-, Piperidin-, 2,2,6,6-Tetramethylpiperidin- oder Morpholinring.

Bevorzugt sind Verbindungen der Formel I, worin m 1 oder 2 ist, X Wasserstoff, $C_1$-$C_8$-Alkyl, Allyl oder Benzyl ist, $R^1$, wenn m 1 ist, $C_1$-$C_{12}$-Alkyl oder eine Gruppe der Formel II ist, und wenn m 2 ist, $C_2$-$C_{10}$-Alkylen oder $C_4$-$C_6$-Oxaalkylen, 1,4-Cyclohexylen, eine Gruppe

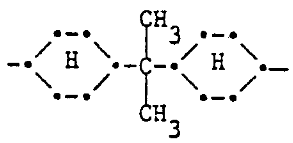

oder

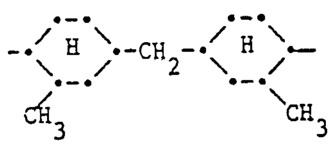

bedeutet und $R^2$ und $R^3$ unabhängig voneinander $C_1$-$C_{10}$-Alkyl, Phenyl, Cyclohexyl, Allyl oder Benzyl bedeuten oder $R^2$ und $R^3$ zusammen $C_4$-$C_6$-Alkylen oder 3-Oxa-1,5-pentylen sind.

Besonders bevorzugt sind Verbindungen der Formel I, worin X Wasserstoff, $C_1$-$C_4$-Alkyl, Allyl oder Benzyl ist, $R^1$, wenn m 1 ist, $C_1$-$C_4$-Alkyl oder eine Gruppe der Formel II ist, und wenn m 2 ist, $C_2$-$C_6$-Alkylen oder $C_4$-$C_6$-Oxaalkylen bedeutet und $R^2$ und $R^3$ $C_1$-$C_{12}$-Alkyl, Phenyl, Cyclohexyl oder Allyl darstellen oder $R^2$ und $R^3$ zusammen $C_4$-$C_6$-Alkylen oder 3-Oxa-1,5-pentylen sind.

Beispiele für Verbindungen der Formel I, worin m 1 ist, sind :

1,1-Dimethyl-3,3-di(2,2,6,6-tetramethylpiperidyl-4)-harnstoff
1,1-Dibutyl-3,3-di(2,2,6,6-tetramethylpiperidyl-4)-harnstoff
1,1-Dibenzyl-3,3-di(1-benzyl-2,2,6,6-tetramethylpiperidyl-4)-harnstoff
1,1,3-Trimethyl-3-(1,2,2,6,6-pentamethylpiperidyl-4)-harnstoff
1,1,3-Triäthyl-3-(2,2,6,6-tetramethylpiperidyl-4)-harnstoff
1,3-Dimethyl-1-octyl-3-(2,2,6,6-tetramethylpiperidyl-4)-harnstoff
1,3-Dimethyl-1-phenyl-3-(2,2,6,6-tetramethylpiperidyl-4)-harnstoff
1,1-Dibutyl-3-cyclohexyl-3-(2,2,6,6-tetramethylpiperidyl-4)-harnstoff
1,2,2,6,6-Pentamethyl-4-[(N-piperidinocarbonyl)-methylamino]-piperidin
1,1-Dimethyl-3,3-di(1-allyl-2,2,6,6-tetramethylpiperidyl-4)-harnstoff

Beispiele für Verbindungen der Formel I, worin m 2 ist, sind :

N,N'-Bis(dimethylcarbamoyl)-N,N'-bis(2,2,6,6-tetramethylpiperidyl-4)-hexamethylendiamin
N,N'-Bis(dimethylcarbamoyl)-N,N'-bis(1-allyl-2,2,6,6-tetramethyl-piperidyl-4)-tetramethylendiamin
N,N'-Bis(diäthylcarbamoyl)-N,N'-bis(1-benzyl-2,2,6,6-tetramethyl-piperidyl-4)-2,2,5-trimethyl-hexamethylendiamin
N,N'-Bis(diäthylcarbamoyl)-N,N'-bis(1,2,2,6,6-pentamethylpiperidyl-4)-dodecamethylendiamin
N,N'-Bis(methyl-phenylcarbamoyl)-N,N'-bis(1,2,2,6,6-pentamethylpiperidyl-4)p-xylylendiamin
$N^1$,$N^4$-Bis(diäthylcarbamoyl)-$N^1$,$N^4$-bis(1,2,2,6,6-pentamethylpiperidyl-4)-$N^2$,$N^3$-dimethyl-triäthylentetramin
1,9-Bis(dicyclohexylcarbamoyl)-1,9-bis(2,2,6,6-tetramethylpiperidyl-4)-5-methyl-1,5,9-triazanonan
1,4,7-Tris(dimethylcarbamoyl)-1,7-bis(2,2,6,6-tetramethylpiperidyl-4)-1,4,7-triazaheptan
N,N'-Bis(dibutylcarbamoyl)-N,N'-bis(2,2,6,6-tetramethylpiperidyl-4)-2,2-di(4-aminocyclohexyl)-propan
N,N'-Bis(diphenylcarbamoyl)-N,N'-bis(1,2,2,6,6-pentamethylpiperidyl-4)-di(4-amino-3-methyl-cyclohexyl)-methan

Die Verbindungen der Formel I können nach verschiedenen Methoden hergestellt werden. Die wichtigste Methode ist die Umsetzung eines 4-Aminopiperidines der Formel III mit einem Carbaminsäurechlorid :

(Siehe Figur Seite 5 f.)

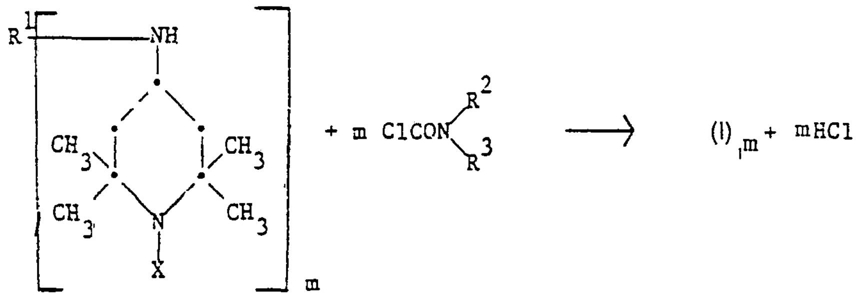


(III)

Die Reaktion wird in Gegenwart von molaren Mengen eines HCl-Akzeptors durchgeführt. Als solche eignen sich anorganische oder organische Basen, beispielsweise Alkalihydroxide, Alkalicarbonate oder tertiäre Amine.

Die Reaktion wird vorzugsweise in einem Lösungsmittel durchgeführt. Als solches eignet sich z. B. Chloroform, Methylenchlorid, Benzol, Toluol, Xylol, Tetrahydrofuran, Dioxan, Dimethoxyäthan oder Dimethylformamid.

Eine zweite Methode ist die Umsetzung von III mit Phosgen, gegebenenfalls in Gegenwart eines Protonakzeptors, gefolgt von der Umsetzung des entstandenen Carbamoylchlorides IV mit einem sekundären Amin :

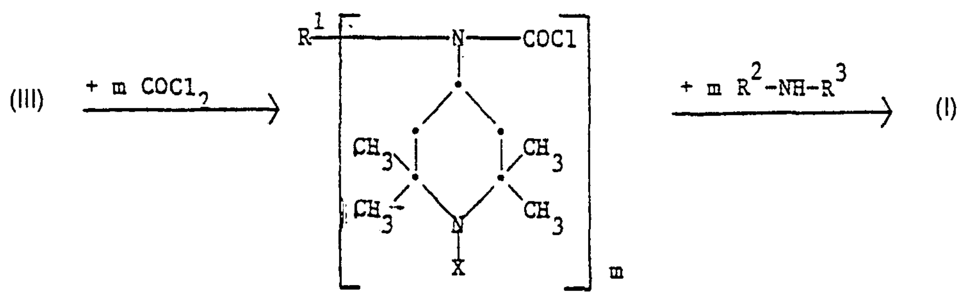


(IV)

Die Umsetzung der ersten Stufe (Phosgenierung) erfolgt in einem inerten Lösungsmittel wie z. B. Benzol, Toluol, Xylol, Chloroform, Aethylacetet etc. bei − 30 bis + 50 °C. Das Zwischenprodukt IV braucht nicht isoliert werden sondern kann unmittelbar nach Beendigung der ersten Stufe mit dem Amin umgesetzt werden. Die Umsetzung mit dem Amin erfolgt unter Zusatz von mindestens 2m Mol HCl-Akzeptor. Es kann hierfür auch ein Ueberschuss des sekundären Amins verwendet werden.

Ein drittes Verfahren besteht in der Umsetzung von III mit einem N,N-disubstituierten Harnstoff :

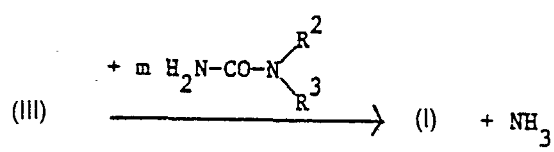


Der Harnstoff kann dabei auch im Ueberschuss verwendet werden. Die Reaktion wird ohne Lösungsmittel oder in einem hochsiedenden, polaren Lösungsmittel wie z. B. Dimethylformamid oder Dimethylsulfoxid ausgeführt bei Temperaturen von 150 bis 250 °C. Der Verlauf der Reaktion kann durch Messung des entstehenden $NH_3$ verfolgt werden.

Die bei allen drei Verfahren als Ausgangsmaterial verwendeten 4-Aminopiperidine der Formel III können aus den entsprechenden 4-Oxopiperidinen durch katalytische Hydrierung in Gegenwart der primären Amine $R^1(NH_2)_n$ gewonnen werden, wie das in den DT-OSS 2 040 975 und 2 349 962 beschrieben ist. Wenn der Substituent X ein Kohlenwasserstoff- oder Acylrest ist, so kann er in die entsprechende NH-Verbindung eingeführt werden durch die üblichen Methoden zur Alkylierung oder Acylierung von sekundären Aminen. Diese Einführung von X kann auf der Stufe der 4-Oxopiperidine geschehen oder — vorzugsweise — auf der Stufe der 4-Ureidopiperidine. Piperidin-1-oxyle (X = O·) können durch Oxydation der 4-Ureido-1-hydrogenpiperidine (Formel I, X = H) mittels Percarbonsäuren oder mittels $H_2O_2$ in Gegenwart von Wolfram-Katalysatoren hergestellt werden.

Eine weitere Möglichkeit zur Herstellung der Verbindungen der Formel I besteht in der N-Alkylierung von tri- oder disubstituierten Harnstoffen der Formel V, VI und VII mit den entsprechenden Alkyl-, Alkenyl- oder Aralkylhalogeniden unter den Bedingungen des Phasentransferverfahrens :

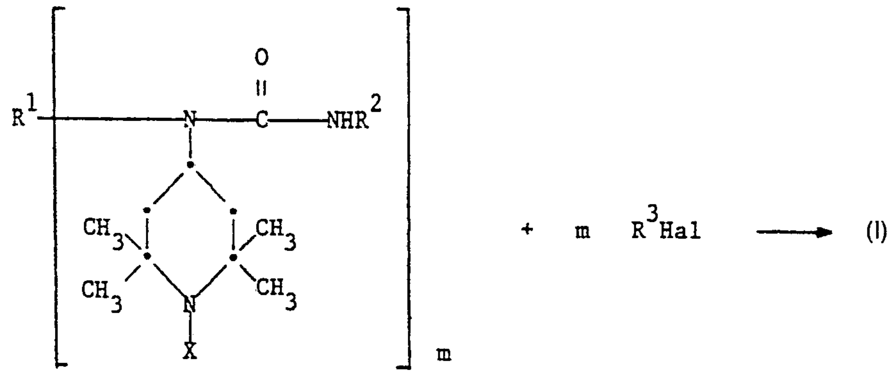

(V)

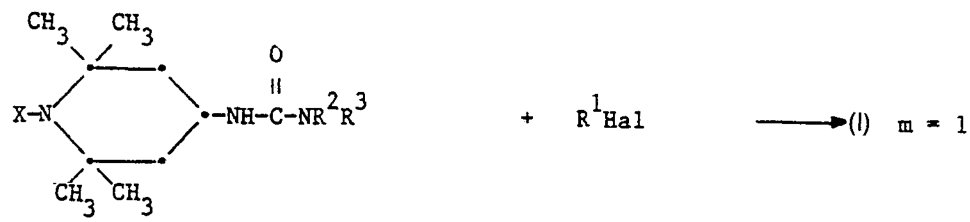


(VI)

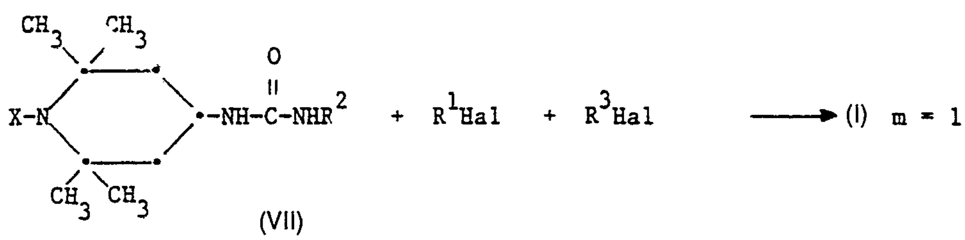


(VII)

Hierbei arbeitet man in einem mit Wasser nicht mischbaren Lösungsmittel wie z. B. Benzol, Toluol, Xylol, Methylenchlorid oder Dioxan und setzt eine der verwendeten Halogenid-Menge entsprechende molare Menge an Alkalihydroxyd in pulverisierter Form oder als konzentrierte wässrige Lösung zu. Auch festes $K_2CO_3$ kann verwendet werden. Weiterhin setzt man katalytische Mengen eines quartären Ammoniumsalzes als Phasentransfer-Katalysator zu, beispielsweise Benzyl-trimethylammoniumchlorid.

Die Verbindungen der Formeln V, VI und VII sind aus den eingangs erwähnten DE-OSS 2 040 975 oder 2 349 962 bekannt oder können nach den dort beschriebenen Methoden hergestellt werden. Vorzugsweise verwendet man solche Verbindungen der Formel V, VI oder VII, worin X kein Wasserstoff ist. Wenn man jedoch eine Verbindung der Formel V, VI oder VII verwendet, worin X Wasserstoff ist, so kann man gleichzeitig den Piperidin-Stickstoff substituieren und man erhält eine Verbindung der Formel I, worin X gleich $R^3$ oder $R^1$ ist. Die Umsetzung einer Verbindung der Formel VII mit $R^1Hal$ und $R^3Hal$ wird vorzugsweise dann verwendet, wenn $R^1$ gleich $R^3$ ist.

Die Verbindungen der Formel I sind meist kristalline Substanzen, die durch Umkristallisation gereinigt werden können. Ihre Schmelzpunkte liegen jedoch niedriger als die der entsprechenden Harnstoffe, die CONH-Gruppen besitzen. Auch ist ihre Löslichkeit und Verträglichkeit in verschiedenen Substanzen höher als die der CONH-Verbindungen.

Die Verbindungen der Formel I zeichnen sich weiterhin durch hervorragende Hydrolysebeständigkeit aus, die die an sich gute Hydrolysebeständigkeit der CONH-Verbindungen noch weit übertrifft.

Die wertvollste Eigenschaft der Verbindungen der Formel I ist ihre hervorragende Stabilisatorwirkung, vor allem gegen den Lichtabbau von organischen Polymeren. Beispiele von Polymeren, die durch Lichteinwirkung geschädigt werden und die durch Zusatz von Verbindungen der Formel I stabilisiert werden können, sind die folgenden Polymeren.

1. Polymere von Mono- und Diolefinen, beispielsweise Polyäthylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyäthylen oder mit Polyisobutylen.

3. Copolymere von Mono- und Diolefinen untereinander oder mit anderen Vinylmonomeren, wie z. B. Aethylen-Propylen-Copolymere, Propylen-Buten-1-Copolymere, Propylen-Isobutylen-Copolymere, Aethy-

len-Buten-1-Copolymere, Propylen-Butadien-Copolymere, Isobutylen-Isopren-Copolymere, Aethylen-Aethylacrylat-Copolymere, Aethylen-Alkylmethacrylat-Copolymere, Aethylen-Vinylacetat-Copolymere oder Aethylen-Acrylsäure-Copolymere und deren Salze (Ionomere), sowie Terpolymere von Aethylen mit Propylen und einem Dien, wie Hexadien, Dicyclopentadien oder Aethylidennorbornen.

4. Polystyrol.

5. Statistische Copolymere von Styrol oder α-Methylstyrol mit Dienen oder Acrylderivaten, wie z. B. Styrol-Butadien, Styrol-Acrylnitril, Styrol-Aethylmethacrylat, Styrol-Acrylnitril-Methacrylat ; Mischungen von hoher Schlagzähigkeit aus Styrol-Copolymeren und einem anderen Polymer wie z. B. einem Polyacrylat, einem Dien-Polymeren oder einem Aethylen-Propylen-Dien-Terpolymeren ; sowie Block-Copolymere des Styrols, wie z. B. Styrol-Butadien-Styrol, Styrol-Isopren-Styrol, Styrol-Aethylen/Butylen-Styrol oder Styrol-Aethylen/Propylen-Styrol.

6. Pfropfcopolymere von Styrol, wie z. B. Styrol auf Polybutadien, Styrol und Acrylnitril auf Polybutadien, Styrol und Alkylacrylate, bzw. Alkylmethacrylate auf Polybutadien, Styrol und Acrylnitril auf Aethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyäthylen, insbesondere Polymere aus halogenhaltigen Vinylverbindungen, wie z. B. Polyvinylchlorid, Polyvinylidenchlorid, Polyvinylfluorid, Polyvinylidenfluorid ; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Vinylchlorid-Copolymere, oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Aethern, wie Polyalkylenglykole, Polyäthylenoxyd, Polypropylenoxyd oder deren Copolymere mit Bisglycidyläthern.

12. Polyacetale, wie Polyoxymethylen, sowie solche Polyoxymethylene, die Comonomere wie z. B. Aethylenoxyd enthalten.

13. Polyphenylenoxyde und -sulfide.

14. Polyurethane, die sich von Polyäthern, Polyestern und Polybutadienen mit endständigen Hydroxylgruppen einerseits und aliphatischen oder aromatischen Polyisocyanaten andererseits ableiten sowie deren Vorsprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Copolymere mit Polyäthern wie z. B. mit Polyäthylenglykol, Polypropylenglykol oder Polytetramethylenglykol.

16. Polyharnstoffe, Polyimide und Polyamid-imide.

17. Polyester, die sich von Dicarbonsäuren und Dialkoholen und/oder von Hydroxycarbonsäuren oder den entsprechenden Lactonen ableiten, wie Polyäthylenterephthalat, Polybutylenterephthalat, Poly-1,4-dimethylolcyclohexanterephthalat, Polyhydroxybenzoate, sowie Block-Polyäther-ester, die sich von Polyäthern mit Hydroxylendgruppen ableiten.

18. Polycarbonate.

19. Polysulfone und Polyäthersulfone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z. B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z. B. von Bis-glycidyläthern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseäther, wie Methylcellulose.

Von besonderer Bedeutung ist die Stabilisierung von Polyolefinen, Styrolpolymerisaten und von Polyurethanen, für die sich die Verbindungen der Formel I hervorragend eignen. Beispiele hierfür sind Polyäthylen hoher und niedriger Dichte. Polypropylen, Aethylen-Propylen-Copolymerisate, Polystyrol, Styrol-Butadien-Acrylnitril-Copolymerisate, Mischungen von Polyolefinen oder von Styrolpolymerisaten, Polyurethane auf Polyäther- oder Polyesterbasis in Form von Filmen, Fasern, Lacken, Elastomeren oder Schaumstoffen. Von besonderer Bedeutung ist weiterhin die Stabilisierung von Lackharzen, z. B. von Alkyd-, Polyester- und Acrylharzen und deren Gemischen mit Melaminharzen.

Die Stabilisatoren werden den Kunststoffen in einer Konzentration von 0,01 bis 5 Gew.-%, berechnet auf das zu stabilisierende Material, zugesetzt. Vorzugsweise werden 0,03 bis 1,5, besonders bevorzugt 0,2 bis 0,6 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Ausser den Verbindungen der Formel I können den Kunststoffen auch noch andere, bekannte Stabilisatoren zugesetzt werden. Dies können z. B. Antioxydantien, Lichtschutzmittel oder Metalldesaktivatoren sein, oder auch Costabilisatoren wie z. B. solche vom Typ der Phosphorigsäureester. Weiterhin können sonstige in der Kunststofftechnologie übliche Zusätze wie z. B. Flammschutzmittel, Antistatika, Weichmacher, Gleitmittel, Treibmittel, Pigmente, Verstärkungsstoffe oder Füllstoffe zugesetzt werden. Einzelne Beispiele solcher bekannter und üblicher Zusätze sind in der DE-OS 2 349 962, Seite 25-32, aufgeführt.

Die Erfindung betrifft daher auch die durch Zusatz von 0,01 bis 5 Gew.-% einer Verbindung der Formel I stabilisierten Kunststoffe, die gegebenenfalls noch andere bekannte und übliche Zusätze enthalten können. Die so stabilisierten Kunststoffe können in verschiedenster Form angewendet werden z. B. als Folien, Fasern, Bändchen, Profile oder als Bindemittel für Lacke, Klebemittel oder Kitte oder als Beschichtung für photographische Filme und Papiere.

Die Herstellung und Verwendung der erfindungsgemässen Verbindungen wird in den folgenden Beispielen näher beschrieben. Teile bedeuten darin Gewichtsteile und % Gewichtsprozente. Die Temperaturen sind in Celsius-Graden angegeben.

## Beispiel 1

Zu einer Lösung von 55 g (0,15 Mol) N,N'-Bis(2,2,6,6-tetramethyl-4-piperidyl)-hexamethylendiamin in 130 ml Xylol und 44,6 g Aethyldiisopropylamin (0,345 mol) tropft man unter Rühren bei 105-110° 33,9 g (0,315 Mol) Dimethylcarbamoylchlorid und rührt insgesamt 12 Std. bei 110°. Zur Aufarbeitung wird das Reaktionsgemisch wiederholt mit Wasser gewaschen, über Natriumsulfat getrocknet und im Vakuum vollständig vom Lösungsmittel befreit. Die rohe Verbindung wird in Ligroin (Sdp. 110-140°) umkristallisiert wodurch N,N'-Bis(dimethylcarbamoyl)-N,N'-bis(2,2,6,6-tetramethylpiperidyl-4) hexamethylendiamin vom Smp. 142-144° erhalten wird.

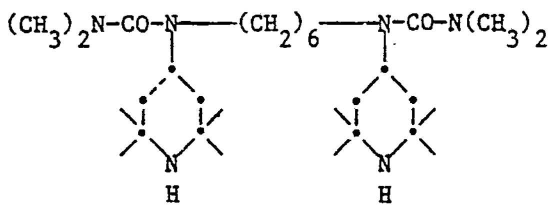

(Verbindung Nr. 1)

In analoger Weise werden die folgenden Verbindungen hergestellt:
N-Bis(2,2,6,6-tetramethylpiperidyl-4)-N'-diäthylharnstoff

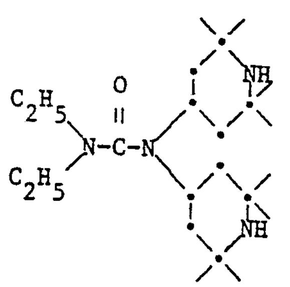

Smp. 154-155°
(Verbindung Nr. 2)

N-Bis(2,2,6,6-tetramethylpiperidyl-4)-N'-dimethylharnstoff

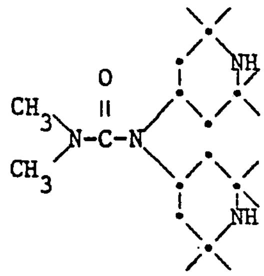

Smp. 137-138°
(Verbindung Nr. 3)

N,N'-Bis(1,2,2,6,6-pentamethylpiperidyl-4)-N,N'-bis(di-sec.butylcarbamoyl)-äthylendiamin

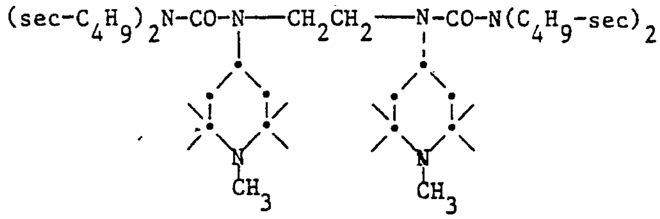

Smp. 194-196°
(Verbindung Nr. 4)

N,N'-Bis(2,2,6,6-tetramethylpiperidyl-4)-N,N'-bis(diphenylcarbamoyl)-hexamethylendiamin

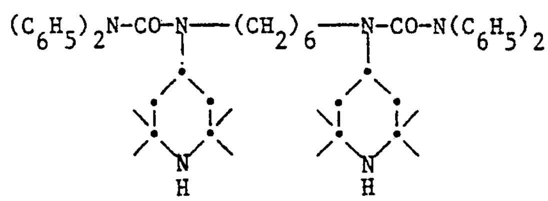

Smp. 250-252°
(Verbindung Nr. 5)

N,N'-Bis(2,2,6,6-tetramethylpiperidyl-4)-N,N'-bis(dimethylcarbamoyl)-1,8-diamino-3,6-dioxa-octan

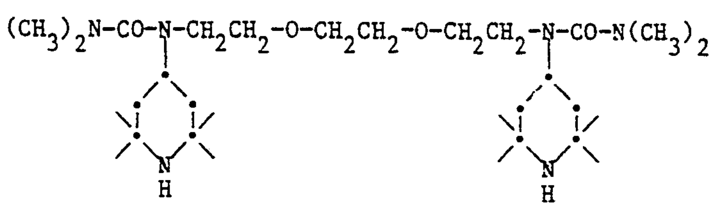

Smp. 70-71°
(Verbindung Nr. 6)

N,N'-Bis(1,2,2,6,6-pentamethylpiperidyl-4)-N,N'-bis(dimethylcarbamoyl)-hexamethylendiamin

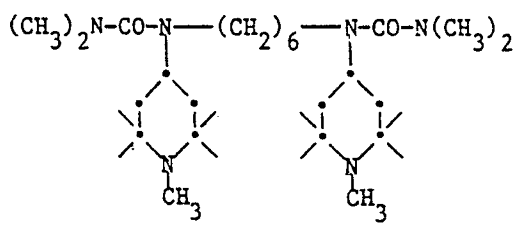

Smp. 136-137°
(Verbindung Nr. 7)

N,N'-Bis(1,2,2,6,6-pentamethylpiperidyl-4)-N,N'-bis(diäthylcarbamoyl)-trimethylendiamin

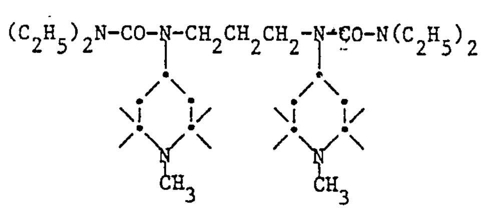

(Verbindung Nr. 8)

viskoses Oel Analyse
Ber: C 68,5 % H 11,5 % N 14,5 %

Gef: C 68,5 % H 11,7 % N 14,5 %

N-Bis(1,2,2,6,6-pentamethylpiperidyl-4)-N'-dimethylharnstoff

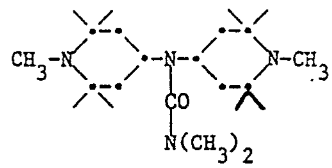

Smp. 156-157°
(Verbindung Nr. 9)

N,N'-Bis(2,2,6,6-tetramethylpiperidyl-4)-N,N'-bis(hexamethyleniminocarbonyl)-hexamethylendiamin

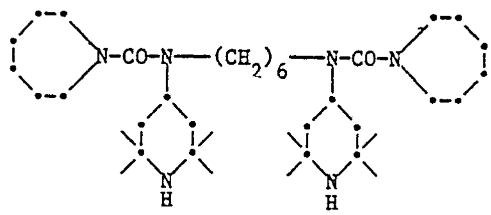

Smp. 98-100°
(Verbindung Nr. 10)

N,N'-Bis(2,2,6,6-tetramethylpiperidyl-4)-N,N'-bis(morpholinocarbonyl)-hexamethylendiamin

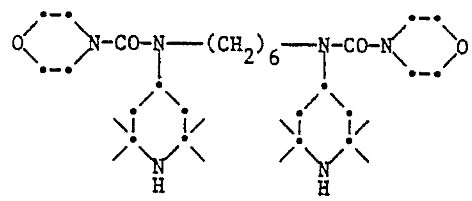

Smp. 123-124°
(Verbindung Nr. 11)

N,N'-Bis(2,2,6,6-tetramethylpiperidyl-4)-N,N'-bis(diallylcarbamoyl)-hexamethylendiamin

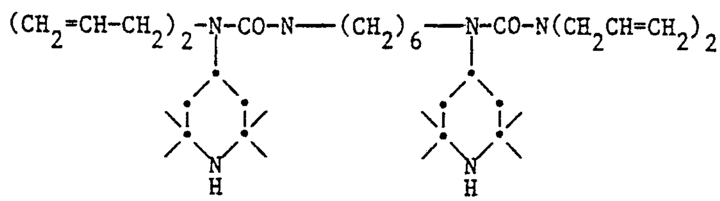

Smp. 73-74°
(Verbindung Nr. 12)

N,N'-Bis(2,2,6,6-tetramethylpiperidyl-4)-N,N'-bis(dicyclohexylcarbamoyl)-hexamethylendiamin

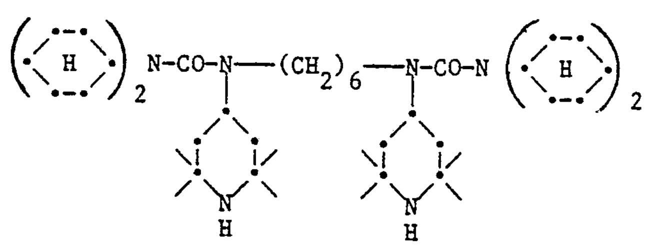

Smp. 216-217°
(Verbindung Nr. 13)

N-Bis(1,2,2,6,6-pentamethylpiperidyl-4)-N'-di-sec.butyl-harnstoff

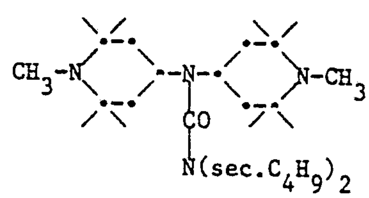

Smp. 100-102°
(Verbindung Nr. 14)

N-Dodecyl-N-(2,2,6,6-tetramethylpiperidyl-4)-N'-dimethylharnstoff

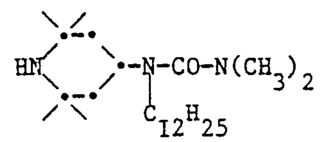

Sdp. 190°/0,03 Torr (Kugelrohr)
(Verbindung Nr. 15)

N-Butyl-N-(1,2,2,6,6-pentamethylpiperidyl-4)-N'-dioctylharnstoff

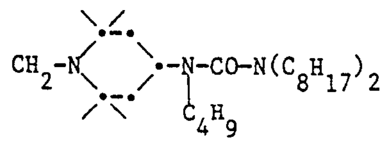

Sdp. 205°/0,01 Torr (Kugelrohr)
(Verbindung Nr. 16)

N,N'-Bis(1,2,2,6,6-pentamethylpiperidyl-4)-N,N'-bis(methylmethoxycarbamoyl)-trimethylendiamin

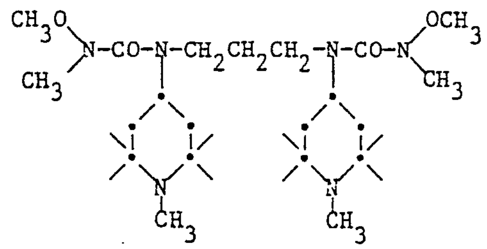

(Verbindung Nr. 17)

Elementar-Analyse : $C_{29}H_{58}N_6O_4$

| | | | |
|---|---|---|---|
| Ber : | C 62,78 % | H 10,54 % | N 15,15 % |
| Gef : | C 62,5 % | H 10,8 % | N 14,9 % |

Beispiel 2

26,9 g (0,05 Mol) N,N'-Bis-dimethylcarbamoyl-N,N'-bis-(2,2,6,6-tetramethyl-4-piperidyl)-hexamethylendiamin (hergestellt nach Beispiel 1), 18,2 g Allylbromid (0,15 Mol), 16,6 g pulverisiertes Kaliumcarbonat, 0,5 g pulverisiertes Kaliumjodid und 70 ml Aethylmethylketon werden 24 Std. bei ca. 72-74° (Rückflusstemperatur) in einer Stickstoffatmosphäre gerührt. Hierauf filtriert man das Reaktionsgemisch von den anorganischen Salzen ab, entfernt das Lösungsmittel im Vakuum und kristallisiert die rohe Verbindung aus Pentan, wodurch N,N'-Bis(dimethylcarbamoyl)-N,N'-bis-(1-allyl-2,2,6,6-tetramethylpiperidyl-4)-hexamethylendiamin vom Smp. 96-98 °C erhalten wird.

Elementar-Analyse : $C_{36}H_{68}N_6O_2$

| | | | | |
|---|---|---|---|---|
| Ber : | C 70,08 % | H 11,11 % | N 13,62 % | O 5,19 % |
| Gef : | C 69,9 % | H 11,3 % | N 13,5 % | O 5,1 % |

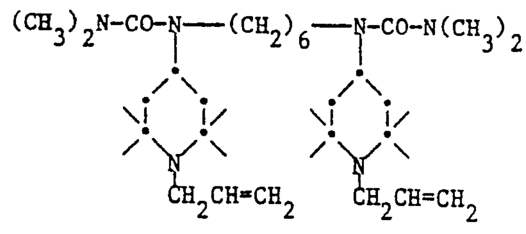

(Verbindung Nr. 18)

In analoger Weise werden hergestellt :
N-Bis(1-allyl-2,2,6,6-tetramethylpiperidyl-4)-N'-dimethylharnstoff

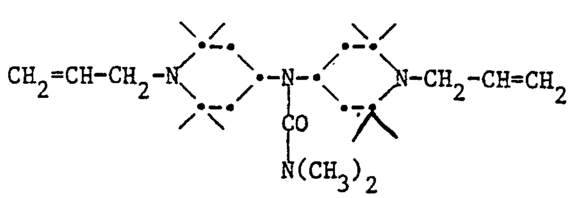

Smp. 88,5-90°
(Verbindung Nr. 19)

N,N'-Bis(1-allyl-2,2,6,6-tetramethylpiperidyl-4)-N,N'-bis(diäthylcarbamoyl)-hexamethylendiamin

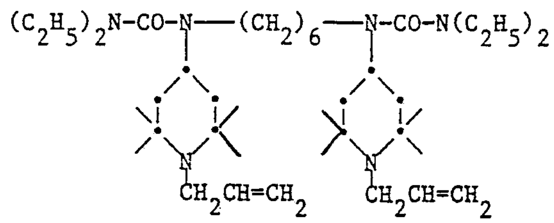

Smp. 68-69°
(Verbindung Nr. 20)

N,N'-Bis(1-allyl-2,2,6,6-tetramethylpiperidyl-4)-N,N'-bis(dicyclohexylcarbamoyl)-hexamethylendiamin

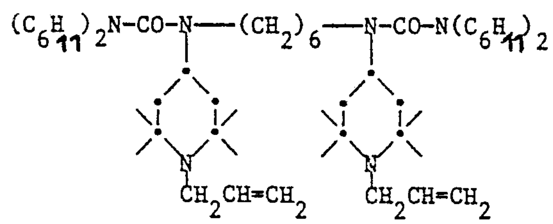

Smp. 172-173°
(Verbindung Nr. 21)

Beispiel 3

Zur einer Lösung von 34,6 g (0,06 Mol) N,N'-Bis(1-benzyl-2,2,6,6-tetramethyl-4-piperidyl)-hexamethylendiamin (Smp : 101-103°, hergestellt durch reduktive Aminierung von 1-Benzyl-2,2,6,6-tetramethyl-4-piperidon mit Hexamethylendiamin) in 150 ml Xylol und 15 g Triäthylamin tropft man bei 110-120° unter Rühren 17,6 g (0,13 Mol) Diäthylcarbamoylchlorid. Nach einer Reaktionszeit von 9 Std. bei ca. 115° wird das Reaktionsgemisch auf Raumtemperatur gekühlt, mit Toluol verdünnt, wiederholt mit Wasser gewaschen, über Natriumsulfat getrocknet und im Vakuum vollständig von den Lösungsmitteln befreit.

Die rohe Verbindung wird durch Chromatographie an Kieselgel (Eluiermittel : Diäthyläther) weiter gereinigt und in n-Hexan umkristallisiert, wodurch reines N,N'-Bis(diäthylcarbamoyl)-N,N'-bis(1-benzyl-2,2,6,6-tetramethyl-4-piperidyl)-hexamethylendiamin erhalten wird. Smp. 123-124°.

Elementar-Analyse : $C_{48}H_{80}N_6O_2$

Ber : C 75,56 % H 10,43 % N 10,87 %

Gef : C 75,7 % H 10,4 % N 10,9 %

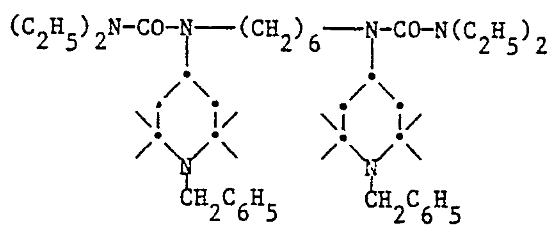

(Verbindung Nr. 22)

Analog wird hergestellt :
N,N'-Bis(2,2,6,6-tetramethylpiperidyl-4)-N,N'-bis(diäthylcarbamoyl)-hexamethylendiamin

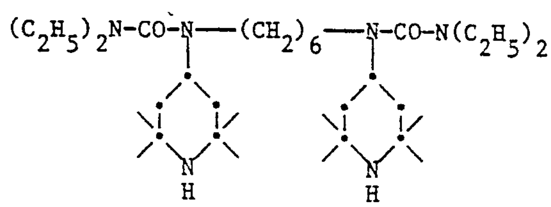

Smp. 142,5-144°
(Verbindung Nr. 23)

Beispiel 4

Zu einem kräftig gerührten Gemisch (unter einer Stickstoff-Atmosphäre) aus 9,65 g N-(1,2,2,6,6-Pentamethylpiperidyl-4)-N'-dimethylharnstoff (hergestellt aus 4-Amino-1,2,2,6,6-pentamethylpiperidin und Dimethylcarbaminsäurechlorid ; Smp. 111-112°), 40 ml Benzol, 5,6 g Natriumhydroxid, 11,06 g Kaliumcarbonat und 1,4 g Tetrabutylammoniumhydrogensulfat tropft man bei 78° innerhalb von 90 Min.

die Lösung von 16,5 g n-Butylbromid in 10 ml Benzol. Anschliessend wird 30 Std. bei 78-80° weitergerührt. Zur Aufarbeitung wird die weisse Suspension auf Raumtemperatur gekühlt, mit 200 ml Diäthyläther versetzt, filtriert, das Filtrat im Vakuum von den Lösungsmitteln befreit und der Rückstand an Kieselgel (60 Merck) säulenchromatographisch gereinigt (Eluierungsmittel: Diäthyläther-Methanol-Triäthylamin 90 : 7 : 3) wodurch reiner N-Butyl-N-(1,2,2,6,6-pentamethylpiperidyl-4)-N'-dimethylharnstoff vom Smp. 83-85° erhalten wird (kristallisiert aus Acetonitril).

$C_{17}H_{35}N_3O$ (281,47)

Ber: C 68,64 % H 11,86 % N 14,13 %

Gef: C 68,5 % H 11,6 % N 14,1 %

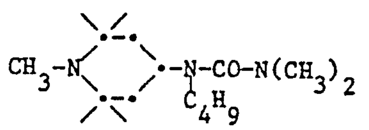

(Verbindung Nr. 24)

Beispiel 5

Zu 40,2 g einer 25,7 %igen Lösung von Phosgen in Xylol (0,105 Mol) tropft man unter Rühren und unter Inertgasatmosphäre innerhalb von 1,5 Std. bei – 40° die Lösung von 29,7 g (0,21 Mol) redestilliertem 2,2,6,6-Tetramethylpiperidin in 50 ml Xylol. Nach der Zugabe des Amins wird 4 Std. bei – 10° und 18 Std. bei – 5 bis 0° weitergerührt. Das so erhaltene 2,2,6,6-Tetramethylpiperidinocarbonsäurechlorid wird nicht isoliert, sondern nun bei – 10° innerhalb von 2 Std. mit einer Lösung von 19,73 g (0,05 Mol) N,N'-Bis(2,2,6,6-tetramethyl-4-piperidyl)-hexamethylendiamin und 20,4 ml (0,12 Mol) Diisopropyl-äthylamin in 50 ml Xylol versetzt. Nach weiteren 18 Std. Rühren bei Raumtemperatur und 6 Std. bei 50 °C wird das Reaktionsgemisch auf Raumtemperatur gekühlt, mit 300 ml Hexan verdünnt, viermal mit 150 ml Wasser extrahiert, die organische Phase über Natriumsulfat getrocknet und die Lösungsmittel im Wasserstrahlvakuum vollständig abdestilliert. Der nach kurzer Zeit kristallin erstarrende Rückstand wird in Acetonitril umkristallisiert, wodurch reines N,N'-Bis(2,2,6,6-tetramethylpiperidyl-4)-N,N'-bis(2,2,6,6-tetramethylpiperidinocarbonyl)-hexamethylendiamin als farblose Verbindung vom Smp. 137-139° erhalten wird.

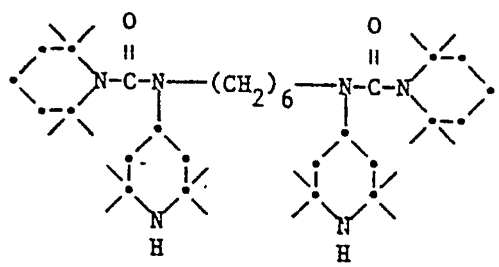

(Verbindung Nr. 25)

Elementaranalyse: $C_{44}H_{84}N_6O_2$

Ber: N 11,54 %

Gef: N 11,5 %

Beispiel 6

Stabilisierung von Polypropylen gegen Licht.

100 Teile Polypropylenpulver (Moplen, Fibre grade, der Firma Montedison) werden mit 0,2 Teilen β-(3,5-Di-tert-butyl-4-hydroxyphenyl)-propionsäure-octadecylester, 0,1 Teilen Calciumstearat und 0,25 Teilen eines Stabilisators der folgenden Tabelle 1 im Brabender-Plastographen bei 200 °C während 10 Minuten homogenisiert. Die so erhaltene Masse wird möglichst rasch dem Kneter entnommen und in einer Kniehebelpresse zu einer 2-3 mm dicken Platte gepresst. Ein Teil des erhaltenen Rohpresslings wird ausgeschnitten und zwischen zwei Hochglanz-Hartaluminiumfolien mit einer handhydraulischen Laborpresse während 6 Minuten bei 260 °C zu einer 0,1 mm dicken Folie gepresst, die unverzüglich in kaltem Wasser abgeschreckt wird. Aus dieser werden nun Abschnitte ausgestanzt und im Xenotest 1 200 belichtet. Zu regelmässigen Zeitabständen werden diese Prüflinge aus dem Belichtungsapparat entnommen und in einem IR-Spektrophotometer auf ihren Carbonylgehalt geprüft. Die Zunahme der Carbonylextinktion bei 5,85 µm während der Belichtung ist ein Mass für den photoxidativen Abbau des Polymeren [s. L. Balaban et al., J. Polymer Sci, Part C ; 22, 1059-1071 (1969)] und ist erfahrungsgemäss mit einem Abfall der mechanischen Eigenschaften des Polymeren verbunden. Als Mass der Schutzwirkung gilt die Zeit bis Erreichen einer Carbonylextinktion von ca. 0,3, bei welcher die Vergleichsfolie brüchig ist.

Das Verhältnis dieser Belichtungszeit zur Belichtungszeit einer Blindprobe ohne Lichtschutzmittel ist der Schutzfaktor SF.

$$SF = \frac{\text{Belichtungszeit Probe}}{\text{Belichtungszeit Blindprobe}}$$

Die folgende Tabelle gibt die Schutzfaktoren der untersuchten Lichtschutzmittel an.

| Verwendetes Lichtschutzmittel | Lichtschutzfaktor SF |
|---|---|
| ohne | 1 |
| Verbindung Nr. 1 | 12 |
| 2 | 13,8 |
| 3 | 15,7 |
| 18 | 8,4 |
| 19 | 13,0 |
| 20 | 8,7 |
| 22 | 6,9 |

**Ansprüche**

1. Verbindungen der Formel I

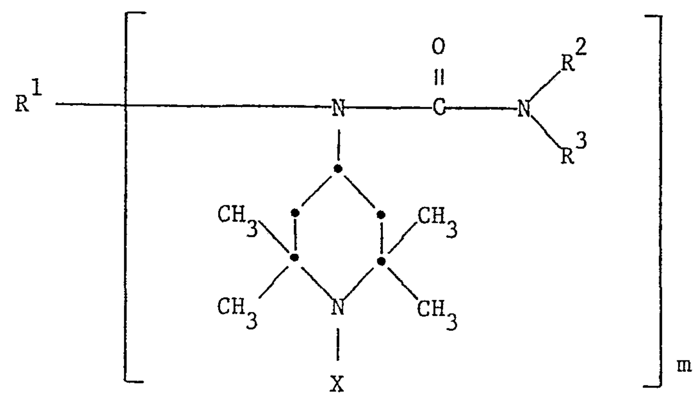

(I)

worin m 1, 2, 3 oder 4 ist, X H, O·, $C_1$-$C_{18}$-Alkyl, $C_3$-$C_5$-Alkenyl, Propargyl, $C_7$-$C_{12}$-Phenylalkyl oder eine Gruppe der Formel —CO—$C_1$-$C_8$-Alkyl, —CO—$C_2$-$C_3$-Alkenyl, —CO—O—$C_1$-$C_{10}$-Alkyl oder —CO—N($C_1$-$C_{10}$-Alkyl)$_2$ bedeutet, $R^1$, wenn m 1 ist, $C_1$-$C_{18}$-Alkyl, $C_3$-$C_5$-Alkenyl, $C_3$-$C_4$-Alkinyl, $C_5$-$C_6$-Cycloalkyl, $C_7$-$C_{18}$-Aralkyl, $C_6$-$C_{12}$-Aryl oder eine Gruppe der Formel II

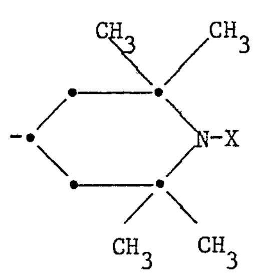

(II)

bedeutet, wenn m 2 ist, $C_2$-$C_{20}$-Alkylen, $C_4$-$C_{12}$-Mono- oder Dioxaalkylen, eine Gruppe $—(CH_2CH_2\underset{R^4}{\underset{|}{N}})_n—CH_2CH_2—$, $—(CH_2)_3—N(R^5)—(CH_2)_3—$, Cyclohexylen, Xylylen, Hexahydroxylylen, oder eine Gruppe

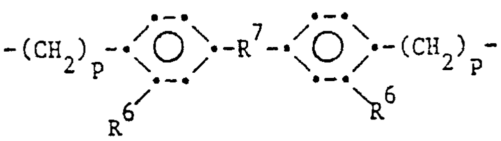 oder 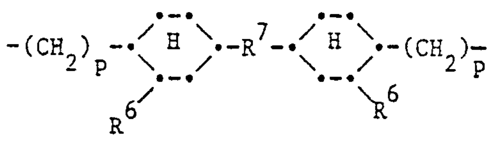

bedeutet, worin $R^4$ Wasserstoff, $C_1$-$C_4$-Alkyl, Allyl, Benzyl oder eine Gruppe

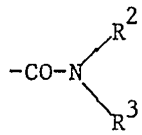

ist, n eine ganze Zahl von 1 bis 5 ist, $R^5$ $C_1$-$C_{12}$-Alkyl, Phenyl oder Cyclohexyl bedeutet, p null oder 1 ist, $R^6$ Wasserstoff oder Methyl ist und $R^7$ $—CH_2—$ oder $>C(CH_3)_2$ bedeutet, wenn m 3 ist, einen Rest

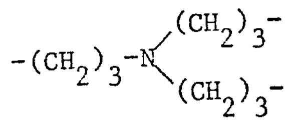

bedeutet, und wenn m 4 ist, einen Rest der Formel

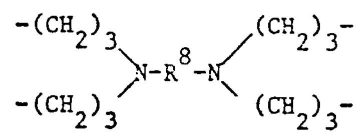

bedeutet, worin $R^8$ $C_2$-$C_{20}$-Alkylen, Cyclohexylen oder Xylylen ist, $R^2$ und $R^3$ unabhängig voneinander $C_1$-$C_{12}$-Alkyl, Cyclohexyl, $C_3$-$C_5$-Alkenyl, Propargyl, $C_7$-$C_{12}$-Phenylalkyl oder $C_6$-$C_{12}$-Aryl bedeuten und $R^2$ auch $C_1$-$C_4$-Alkoxy bedeuten kann oder $R^2$ und $R^3$ zusammen $C_4$-$C_9$-Alkylen oder 3-Oxa-1,5-pentylen bilden.

2. Verbindungen gemäss Anspruch 1 der Formel I, worin m 1 oder 2 ist, X Wasserstoff, $C_1$-$C_8$-Alkyl, Allyl oder Benzyl ist, $R^1$, wenn m 1 ist, $C_1$-$C_{12}$-Alkyl oder eine Gruppe der Formel II ist, und wenn m 2 ist, $C_2$-$C_{10}$-Alkylen oder $C_4$-$C_6$-Oxaalkylen, 1,4-Cyclohexylen, eine Gruppe

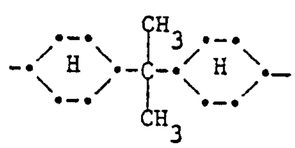 oder 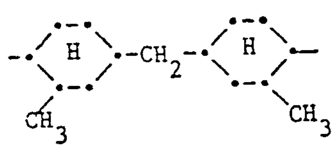

bedeutet und $R^2$ und $R^3$ unabhängig voneinander $C_1$-$C_{10}$-Alkyl, Phenyl, Cyclohexyl, Allyl oder Benzyl darstellen oder $R^2$ und $R^3$ zusammen $C_4$-$C_6$-Alkylen oder 3-Oxa-1,5-pentylen sind.

3. Verbindungen gemäss Anspruch 1 der Formel I, worin m 1 oder 2 ist, X Wasserstoff, $C_1$-$C_4$-Alkyl, Allyl oder Benzyl ist, $R^1$, wenn m 1 ist, $C_1$-$C_4$-Alkyl oder eine Gruppe der Formel II ist, und wenn m 2 ist, $C_2$-$C_6$-Alkylen oder $C_4$-$C_6$-Oxaalkylen bedeutet und $R^2$ und $R^3$ $C_1$-$C_{12}$-Alkyl, Phenyl, Cyclohexyl oder Allyl darstellen oder $R^2$ und $R^3$ zusammen $C_4$-$C_6$-Alkylen oder 3-Oxa-1,5-pentylen sind.

4. Die Verbindung gemäss Anspruch 1, N-Bis(2,2,6,6-tetramethylpiperidyl-4)-N'-dimethylharnstoff.

5. Die Verbindung gemäss Anspruch 1, N-Dodecyl-N-(2,2,6,6-tetramethylpiperidyl-4)-N'-dimethylharnstoff.

6. Die Verbindung gemäss Anspruch 1, N,N'-Bis(2,2,6,6-tetramethylpiperidyl-4)-N,N'-bis(dimethylcarbamoyl)-hexamethylendiamin.

7. Verwendung von Verbindungen der Formel I des Anspruches 1 als Stabilisatoren für Polymere.

8. Gegen Lichteinwirkung stabilisierte Polymerzusammensetzung, gekennzeichnet durch einen Gehalt von 0,01 bis 5 Gewichts-% mindestens einer Verbindung der Formel I des Anspruches 1.

9. Polymerzusammensetzung gemäss Anspruch 8, dadurch gekennzeichnet, dass das Polymer ein Polyolefin, ein Styrolpolymerisat oder ein Polyurethan ist.

10. Polymerzusammensetzung gemäss Anspruch 8, dadurch gekennzeichnet, dass das Polymer ein Lackharz ist.

**Claims**

1. A compound of the formula I

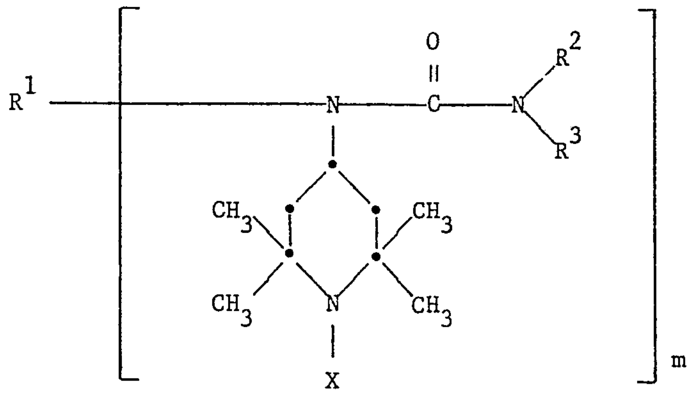

(I)

in which m is 1, 2, 3 or 4, X is H, O·, $C_1$-$C_{18}$-alkyl, $C_3$-$C_5$-alkenyl, propargyl, $C_7$-$C_{12}$-phenylalkyl or a group of the formula —CO—$C_1$-$C_8$-alkyl, —CO—$C_2$-$C_3$-alkenyl, —CO—O—$C_1$-$C_{10}$-alkyl or —CO—N—($C_1$-$C_{10}$-alkyl)$_2$, and $R^1$, if m is 1, is $C_1$-$C_{18}$-alkyl, $C_3$-$C_5$-alkenyl, $C_3$-$C_4$-alkynyl, $C_5$-$C_6$-cycloalkyl, $C_7$-$C_{18}$-aralkyl, $C_6$-$C_{12}$-aryl or a group of the formula II

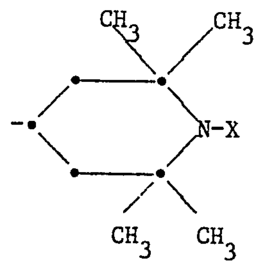

(II)

or, if m is 2, is $C_2$-$C_{20}$-alkylene, $C_4$-$C_{12}$-mono- or dioxa-alkylene, a $-(CH_2CH_2\underset{R^4}{N})_n-CH_2CH_2-$ or $-(CH_2)_3-N(R^5)-(CH_2)_3-$ group, cyclohexylene, xylylene, hexahydroxylylene or a

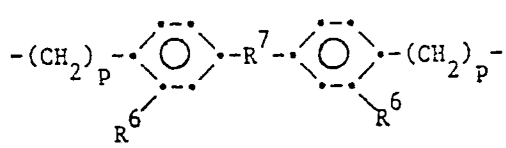 or 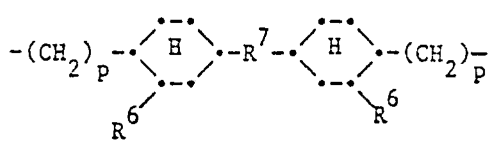

group, in which $R^4$ is hydrogen, $C_1$-$C_4$-alkyl, allyl, benzyl or a

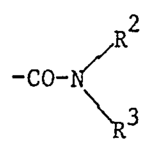

group, n is an integer from 1 to 5, $R^5$ is $C_1$-$C_{12}$-alkyl, phenyl or cyclohexyl, p is nought or 1, $R^6$ is hydrogen or methyl and $R^7$ is $-CH_2-$ or $=C(CH_3)_2$ or $R^1$, if m is 3, is a

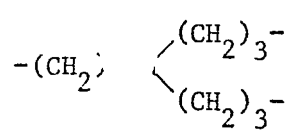

radical and if m is 4 is a radical of the formula

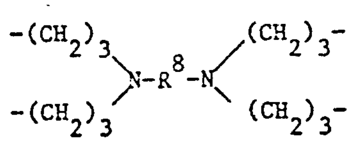

in which $R^8$ is $C_2$-$C_{20}$-alkylene, cyclohexylene or xylylene, and $R^2$ and $R^3$ independently of one another are $C_1$-$C_{12}$-alkyl, cyclohexyl, $C_3$-$C_5$-alkenyl, propargyl, $C_7$-$C_{12}$-phenylalkyl or $C_6$-$C_{12}$-aryl, and $R^2$ can also be $C_1$-$C_4$-alkoxy, or $R^2$ and $R^3$ together form $C_4$-$C_9$-alkylene or 3-oxa-1,5-pentylene.

2. A compound according to claim 1, of the formula I, in which m is 1 or 2, X is hydrogen, $C_1$-$C_8$-alkyl, allyl or benzyl and $R^1$, if m is 1, is $C_1$-$C_{12}$-alkyl or a group of the formula II, and, if m is 2, is $C_2$-$C_{10}$-alkylene or $C_4$-$C_6$-oxaalkylene, 1,4-cyclohexylene or a

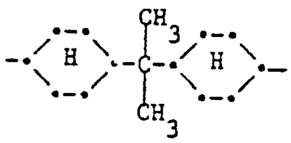

or

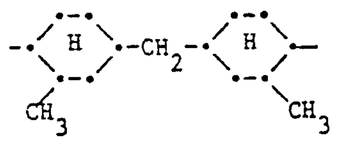

group, and $R^2$ and $R^3$ independently of one another are $C_1$-$C_{10}$-alkyl, phenyl, cyclohexyl, allyl or benzyl, or $R^2$ and $R^3$ together are $C_4$-$C_6$-alkylene or 3-oxa-1,5-pentylene.

3. A compound according to claim 1, of the formula I, in which m is 1 or 2, X is hydrogen, $C_1$-$C_4$-alkyl, allyl or benzyl and $R^1$, if m is 1, is $C_1$-$C_4$-alkyl or a group of the formula II, and if m is 2 is $C_2$-$C_6$-alkylene or $C_4$-$C_6$-oxaalkylene, and $R^2$ and $R^3$ are $C_1$-$C_{12}$-alkyl, phenyl, cyclohexyl or allyl, or $R^2$ and $R^3$ together are $C_4$-$C_6$-alkylene or 3-oxa-1,5-pentylene.

4. N-Bis(2,2,6,6-tetramethylpiperid-4-yl)-N'-dimethylurea according to claim 1.

5. N-Dodecyl-N-(2,2,6,6-tetramethylpiperid-4-yl)-N'-dimethylurea according to claim 1.

6. N,N'-Bis(2,2,6,6-tetramethylpiperid-4-yl)-N,N'-bis(dimethylcarbamoyl)hexamethylenediamine.

7. Use of a compound of the formula I according to claim 1 as light stabiliser for polymers.

8. A polymer composition stabilised against the action of light, the said composition containing 0.01 to 5 % by weight of at least one compound of the formula I of claim 1.

9. A polymer composition according to claim 8, wherein the polymer is a polyolefin, a styrene polymer or a polyurethane.

10. A polymer composition according to claim 8, wherein the polymer is a lacquer resin.

**Revendications**

1. Composés répondant à la formule I

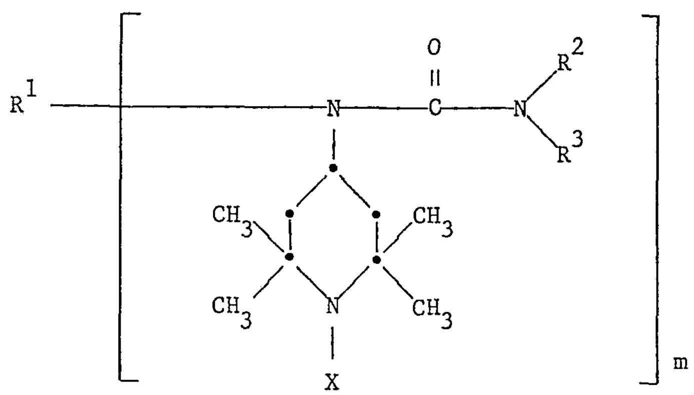

(I)

dans laquelle m est égal à 1, à 2, à 3 ou à 4, X représente H, O·, un alkyle en $C_1$-$C_{18}$, un alcényle en $C_3$-$C_5$, un propyne-2 yle (propargyle), un phénylalkyle en $C_7$-$C_{12}$ ou un radical —CO—$C_1$-$C_8$-Alkyl, —CO—$C_2$-$C_3$-Alcényl, —CO—O—$C_1$-$C_{10}$-Alkyl ou —CO—N($C_1$-$C_{10}$-Alkyl)$_2$, $R^1$ représente, lorsque m est égal à 1, un alkyle en $C_1$-$C_{18}$, un alcényle en $C_3$-$C_5$, un alcynyle en $C_3$ ou $C_4$, un cycloalkyle en $C_5$ ou $C_6$, un aralkyle en $C_7$-$C_{18}$, un aryle en $C_6$-$C_{12}$ ou un radical de formule II

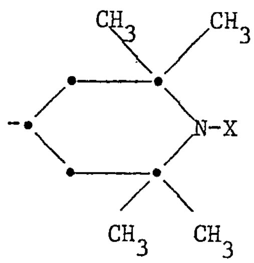

(II)

lorsque m est égal à 2, un alkylène en $C_2$-$C_{20}$, un mono- ou dioxa-alkylène en $C_4$-$C_{12}$, un radical

$-(CH_2CH_2\underset{R^4}{N})-_nCH_2CH_2-$, $-(CH_2)_3-N(R^5)-(CH_2)_3-$, cyclohexylène, xylylène, hexahydroxylylène ou un radical

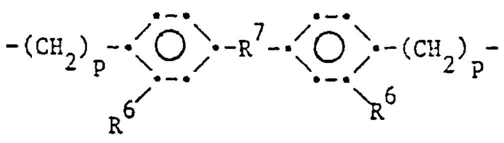

ou

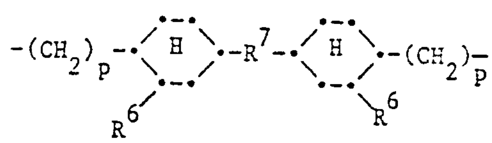

radicaux dans lesquels $R^4$ représente l'hydrogène, un alkyle en $C_1$-$C_4$, un allyle, un benzyle ou un radical

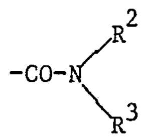

n désigne un nombre entier de 1 à 5, $R^5$ représente un alkyle en $C_1$-$C_{12}$, un phényle ou un cyclohexyle, p est égal à 0 ou à 1, $R^6$ représente l'hydrogène ou un méthyle et $R^7$ représente $-CH_2-$ ou $>C(CH_3)_2$, lorsque m est égal à 3, un radical

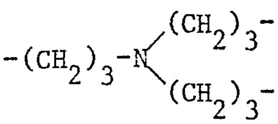

et lorsque m est égal à 4, un radical répondant à la formule

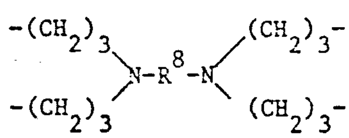

dans laquelle $R^8$ représente un alkylène en $C_2$-$C_{20}$, un cyclohexylène ou un xylylène, $R^2$ et $R^3$ représente chacun, indépendamment l'un de l'autre, un alkyle en $C_1$-$C_{12}$, un cyclohexyle, un alcényle en $C_3$-$C_5$, un propargyle, un phénylalkyle en $C_7$-$C_{12}$ ou un aryle en $C_6$-$C_{12}$, $R_2$ peut aussi représenter un alcoxy en $C_1$-$C_4$, ou encore $R_2$ et $R_3$ peuvent former ensemble un alkylène en $C_4$-$C_9$ ou un oxa-3 pentylène-1,5.

2. Composés de formule I selon la revendication 1 dans lesquels m est égal à 1 ou à 2, X représente l'hydrogène, un alkyle en $C_1$-$C_8$, un alkyle ou un benzyle, $R^1$ représente, lorsque m est égal à 1, un alkyle en $C_1$-$C_{12}$ ou un radical de formule II, et, lorsque m est égal à 2, un alkylène en $C_2$-$C_{10}$, un oxa-alkylène en $C_4$-$C_6$, un cyclohexylène-1,4, un radical

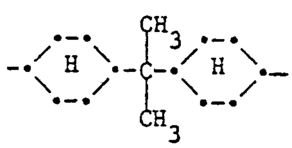

ou

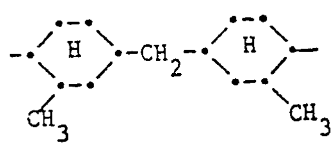

et $R^2$ et $R^3$ représentent chacun, indépendamment l'un de l'autre, un alkyle en $C_1$-$C_{10}$, un phényle, un cyclohexyle, un allyle ou un benzyle ou forment ensemble un alkylène en $C_4$-$C_6$ ou un oxa-3 pentylène-1,5.

3. Composés de formule I selon la revendication 1 dans lesquels m est égal à 1 ou à 2, X représente l'hydrogène, un alkyle en $C_1$-$C_4$, un allyle ou un benzyle, $R^1$ représente, lorsque m est égal à 1, un alkyle en $C_1$-$C_4$ ou un radical de formule II et, lorsque m est égal à 2, un alkylène en $C_2$-$C_6$ ou un oxa-alkylène en $C_4$-$C_6$, et $R^2$ et $R^3$ représentent chacun un alkyle en $C_1$-$C_{12}$, un phényle, un cyclohexyle ou un allyle ou forment ensemble un alkylène en $C_4$-$C_6$ ou un oxa-3 pentylène-1,5.

4. Composé selon la revendication 1, en l'espèce la N-bis-(tétraméthyl-2,2,6,6 pipéridyl-4)-N'-diméthyl-urée.

5. Composé selon la revendication 1, en l'espèce la N-dodécyl-N-(tétraméthyl-2,2,6,6 pipéridyl-4)-N'-diméthyl-urée.

6. Composé selon la revendication 1, en l'espèce la N,N'-bis-(tétraméthyl-2,2,6,6 pipéridyl-4)-N,N'-bis-(diméthylcarbamoyl)-hexaméthylène-diamine.

7. Application de composés de formule I selon la revendication 1 comme stabilisants pour polymères.

8. Composition à base d'un polymère qui a été stabilisée contre l'action de la lumière, composition caractérisée en ce qu'elle contient de 0,01 à 5 % en poids d'au moins un composé de formule I selon la revendication 1.

9. Composition à base d'un polymère selon la revendication 8, caractérisée en ce que le polymère est une polyoléfine, un polymère du styrène ou un polyuréthanne.

10. Composition à base d'un polymère selon la revendication 8, caractérisée en ce que le polymère est une résine pour vernis et peinture.